# EUROPEAN PATENT APPLICATION

(11) **EP 3 995 180 A1**
(43) Date of publication of application: **11.05.2022**
(21) Application number: 20834755.9
(22) Date of filing: 27.05.2020
(51) Int. Cl.: A61Q 19/00, A61K 8/02, A61K 8/31, A61K 8/81, A61K 8/85, A61K 8/89

(54) **MOISTURIZING FILM COSMETIC MATERIAL**

(30) Priority: 03.07.2019 JP 2019124746
(71) Applicant: Shiseido Company, Ltd., Chuo-ku, Tokyo 104-0061 (JP)
(72) Inventor: SOGABE, Atsushi, Tokyo 104-0061 (JP)
(74) Representative: Cabinet Beau de Loménie
(86) International application number: PCT/JP2020/020988
(87) International publication number: WO 2021/002124

(57) **Abstract**

There is provided a cosmetic which can moisturize the skin and exhibit a beautifying effect in low humidity environments while reducing sticky uncomfortable feelings in high humidity environments or sweaty states. A moisturizing film cosmetic of the present disclosure comprises a polyvinyl alcohol film

## Description

### FIELD

The present disclosure relates to a moisturizing film cosmetic.

### BACKGROUND

In recent years, films having water-vapor permeability have been used in various fields.

Patent Literature 1 discloses a water-vapor-permeable film which contains collagen powder composed of a matrix resin and crosslinked regenerated collagen, wherein the water-vapor-permeable film has a communication degree of 1 to 95% and is used in clothing.

Patent Literature 2 discloses a wound bed environment adjustment sheet which is applied to the affected area of skin wounds and in which a water-vapor-permeable hydrogel sheet is used.

### [CITATION LIST]

### [PATENT LITERATURE]

[PTL 1] Japanese Unexamined Patent Publication (Kokai) No. 2010-077202
[PTL 2] WO 2012/036064

### SUMMARY

### [TECHNICAL PROBLEM]

For example, in the case of Japan, while the humidity of the outdoor environment is high during the rainy season, when there is significant rain, the humidity tends to be considerably low during the winter when there is little rain. Furthermore, in recent years, due to the use of air conditioners and the like, the humidity may be considerably low even in indoor work environments or shopping centers. In such low humidity environments, the moisture from the skin evaporates and the skin loses its moisture, whereby skin issues such as rough skin and wrinkling tend to occur.

In order to improve such skin issues, moisturizers such as glycerin are generally applied to the skin. However, even if the moisturizing component applied to the skin temporarily retains the moisture generated from the skin and exhibits moisturizing performance, when the humidity of the outside air is low, the retained moisture evaporates into the outside air over time, whereby a sufficient moisturizing effect cannot be obtained. Furthermore, if an individual sweats while such a moisturizer is applied to their skin, due to the occlusion effect of the applied moisturizer, it becomes difficult for sweat to evaporate into the outside air, whereby a sticky uncomfortable feeling can occur.

Note that when a gel sheet having water-vapor permeability, as described in Patent Literature 2, is applied to the skin, sweat can easily evaporate into the outside air, and it is considered that the moisturizing performance is improved as compared with the case where such a sheet is not applied to the skin. However, since such sheets always exhibit breathability, in low humidity environments, the moisture from the skin continuously evaporates through the sheet. Thus, even when a sheet having such water-vapor permeability is used, in low humidity environments where moisture from the skin can easily evaporate, it is considered that a sufficient moisturizing effect cannot be obtained and skin issues such as rough skin cannot be sufficiently improved.

Thus, an object of the present disclosure is to provide a cosmetic which can moisturize the skin and exhibit a beautifying effect in low humidity environments while reducing sticky uncomfortable feelings in high humidity environments or sweaty states.

### [SOLUTION TO PROBLEM]

### <Aspect 1>

A moisturizing film cosmetic, comprising a polyvinyl alcohol film.

### <Aspect 2>

The cosmetic according to Aspect 1, wherein the thickness of the polyvinyl alcohol film is 100 µm or less.

### <Aspect 3>

The cosmetic according to Aspect 1 or 2, wherein the polyvinyl alcohol film contains an unmodified polyvinyl alcohol.

### <Aspect 4>

The cosmetic according to any one of Aspects 1 to 3, further comprising a waterproof and water-vapor-permeable layer arranged closer to a body surface side than the polyvinyl alcohol film.

### <Aspect 5>

The cosmetic according to Aspect 4, wherein the waterproof and water-vapor-permeable layer is at least one selected from an oil layer containing at least one of petroleum jelly and liquid paraffin, a polylactic acid layer, and an artificial skin.

### <Aspect 6>

The cosmetic according to any one of Aspects 1 to 5, wherein, when evaluated by a water evaporation test, an amount of water evaporation as measured in a 40% RH environment at 23 °C is less than an amount of water evaporation as measured in a 70% RH environment at 28 °C.

### <Aspect 7>

A composition for producing a polyvinyl alcohol film of the cosmetic according to any one of Aspects 1 to 6, comprising a polyvinyl alcohol and water.

### [ADVANTAGEOUS EFFECTS OF INVENTION]

According to the present disclosure, a cosmetic which can moisturize the skin and exhibit a beautifying effect in low humidity environments while reducing sticky uncomfortable feelings in high humidity environments or sweaty states can be provided.

### BRIEF DESCRIPTION OF DRAWINGS

FIG. 1 is a schematic view showing a moisture shielded state in the case in which the moisturizing film cosmetic according to the present disclosure is applied to skin in a low humidity environment.
FIG. 2 is a schematic view showing a moisture permeable state in the case in which the moisturizing film cosmetic according to the present disclosure is applied to skin in a high humidity environment.
FIG. 3 is a schematic view showing a moisture permeable state in the case in which a thin moisturizing film cosmetic according to the present disclosure is applied to skin having a comparatively high moisture content in a low humidity environment.
FIG. 4 is a schematic view showing a moisture shielded state in the case in which a waterproof and water-vapor-permeable layer and a thin moisturizing film cosmetic according to the present disclosure are applied to skin having a comparatively high moisture content in a low humidity environment.
FIG. 5 is a schematic view of a measurement sample which is sealed with a polyvinyl alcohol film used in a water evaporation test.
FIG. 6 is a graph related to the amount of water evaporation of each sample in a water evaporation test in 28 °C 70% RH (high humidity environment) and 23 °C 40% RH (low humidity environment) environments.

### DESCRIPTION OF EMBODIMENTS

The embodiments of the present disclosure will be described below. The present disclosure is not limited to the following embodiments, and various changes can be made within the scope of the gist of the invention.

The moisturizing film cosmetic of the present disclosure comprises a polyvinyl alcohol film.

Without being limited by theory, it is considered that the principle of action by which the cosmetic comprising a polyvinyl alcohol film can moisturize the skin and exhibit a beautifying effect in low humidity environments while reducing sticky uncomfortable feelings in high humidity environments or sweaty states is as described below. Note that in the present disclosure, polyvinyl alcohol is referred to as "PVA" in some cases.

Skin which has been exposed to dryness may be unknowingly deprived of moisture, resulting in an inability to maintain the moisture of the horny layer of the skin surface. When the skin is moisture-deficient, the moisturizing component (Natural Moisturizing Factor (NMF)) produced by the skin itself cannot be sufficiently produced. As a result, the barrier function and the moisturizing function on the surface of the skin are reduced, whereby the skin can easily be damaged, and the skin loses moisture, and it is considered that rough skin is caused thereby.

If the horny layer on the skin surface continues to lack moisture, as new cells are created quickly to repair the damaged horny layer, the speed of skin regeneration (turnover) increases, and at the same time, immature and incomplete cells are increasingly produced. As a result, it is believed that the preparation for forming a normal horny layer becomes insufficient, the skin surface does not retain sufficient moisture, and the skin becomes rough, resulting in a vicious cycle.

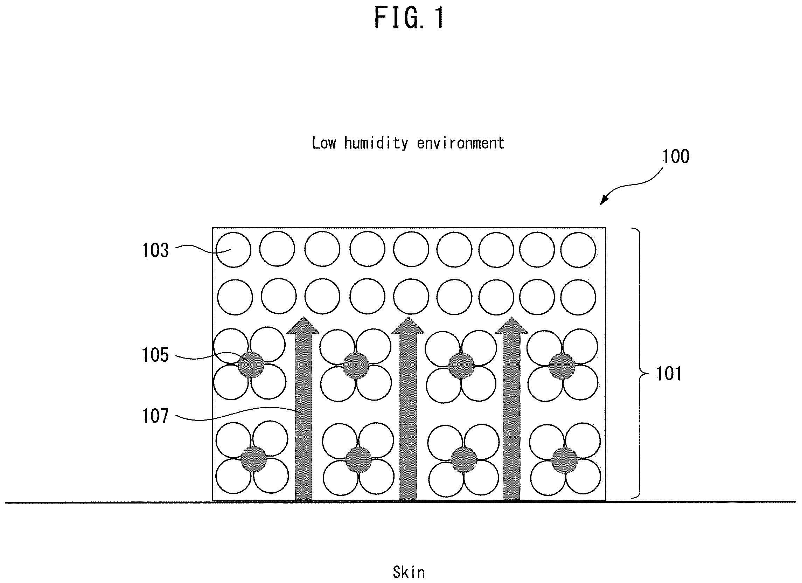

It is generally known that PVA has an excellent affinity with moisture. For example, as shown in FIG. 1, when a PVA film 101 is applied to the skin, it is considered that moisture 105 evaporated from the skin binds to PVA molecules 103 in the film, and the PVA molecules 103 move toward and are attracted by the moisture 105, resulting in gaps 107 through which the moisture passes. However, when the humidity of the external environment is low, the PVA molecules 103 do not move near the outermost surface of the PVA film 101, and it is considered that gaps 107 through which moisture passes are not formed. As a result, in low humidity environments, since the moisture evaporated from the skin stays below the outermost surface of the PVA film and is not evaporated into the atmosphere, it is considered that the skin can be sufficiently moisturized even in low humidity environments. As a result, the function of producing the moisturizing component created by the skin itself is improved, and turnover disorders in the horny layer are also improved. Therefore, it is considered that skin troubles such as rough skin are less likely to occur and the beautifying effect is enhanced.

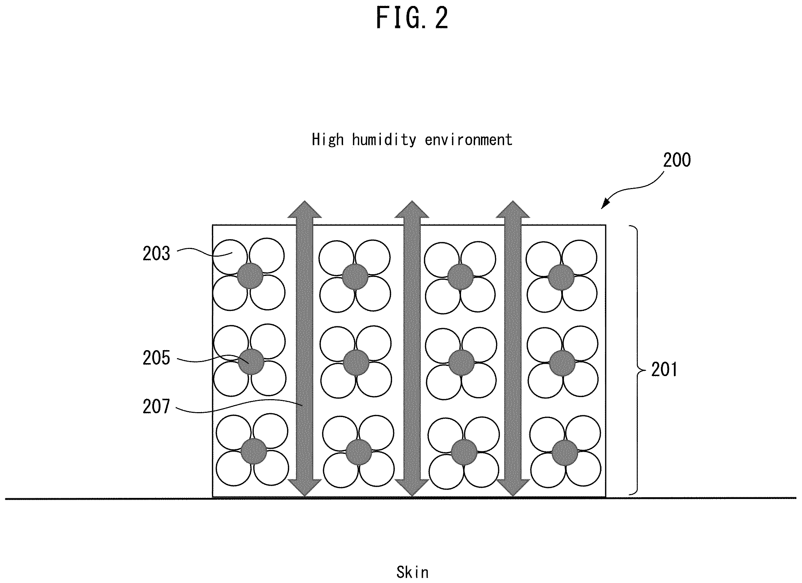

Conversely, in high humidity environments or in a state of excess sweat, for example, as shown in FIG. 2, PVA molecules 203 near the outermost surface of PVA film 201 also move toward and are attracted by moisture 205, and it is considered that gaps 207 through which the moisture passes are generated in the entire area of the film in the thickness direction. As a result, under such circumstances, excess moisture such as sweat can be evaporated from the skin, and it is considered that the sticky uncomfortable feeling can be reduced. Further, when the skin is dry in high humidity environments, it is considered that the moisture in the environment can be provided to the skin through the PVA film 201.

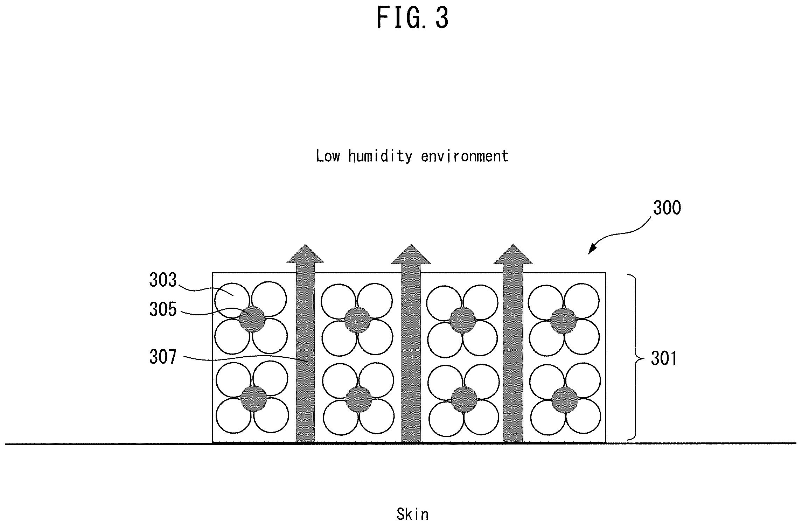

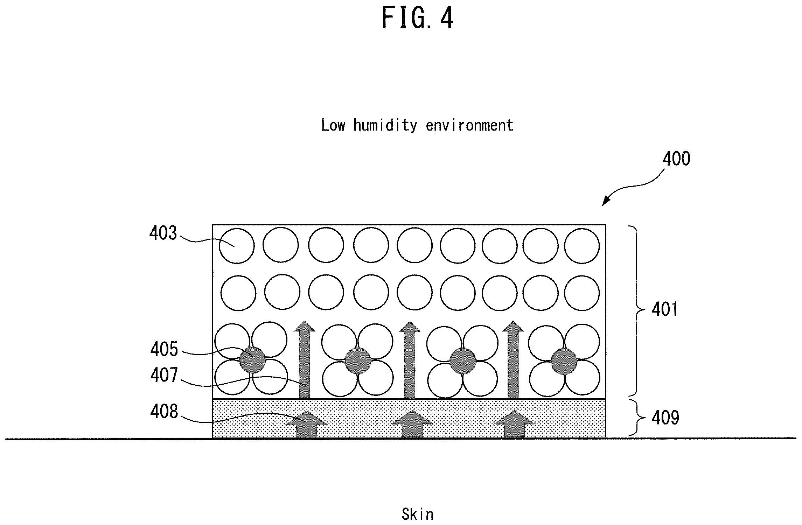

As shown in FIG. 3, for example, when PVA film 301 is thin and the amount of moisture on the skin is large, gaps 307 through which the moisture passes are formed even in low humidity environments, and the moisture from the skin may evaporate. In such a case, as shown in FIG. 4, by applying a waterproof and water-vapor-permeable layer 409 between PVA film 401 and the skin, the formation of gaps 407 through which the moisture passes can be suppressed. Though the waterproof and water-vapor-permeable layer 409 has breathability, it suppresses the movement of moisture from bare skin. Thus, moisture 408 passing through the waterproof and water-vapor-permeable layer 409 invades the PVA film 401 at a smaller ratio than in the case in which the waterproof and water-vapor-permeable layer 409 is not present, whereby even in the above case, it is considered that the gaps 407 through which moisture passes due to the movement of the PVA molecule 403 are not formed near the outermost surface of the PVA film 401, and the skin can be moisturized.

The definitions of the terms used in the present disclosure are as follows.

As used herein, "humidity responsiveness" means the performance of changing the amount of evaporation of moisture in response to changes in the humidity environment, and specifically, the performance of reducing the amount of water evaporation in low humidity environments to less than the amount of water evaporation in high humidity environments.

As used herein, "low humidity environments" means environments of relative humidity of 50% RH or less, 45% RH or less, 40% RH or less, 35% RH or less, or 30% RH or less at atmospheric pressure and 23 °C. The lower limit of such relative humidity is not particular limited, and can be, for example, 3% RH or more, 5% RH or more, 7% RH or more, or 10% RH or more. Furthermore, such relative humidity can be converted to, for example, a weight absolute humidity that is not influenced by temperature. The weight absolute humidity can be 8.7 g/kg or less, 7.9 g/kg or less, 7.0 g/kg or less, 6.1 g/kg or less, or 5.2 g/kg or less, and can be 0.52 g/kg or more, 0.86 g/kg or more, 1.2 g/kg or more, or 1.7 g/kg or more.

As used herein, "high humidity environments" means environments of relative humidity of 50% RH or more, 55% RH or more, 60% RH or more, 65% RH or more, 70% RH or more, or 75% RH or more at atmospheric pressure and 28 °C. The upper limit of such relative humidity is not particular limited, and can be, for example, 100% RH or less or less than 100% RH. Furthermore, such relative humidity can be converted to, for example, a weight absolute humidity that is not influenced by temperature. The weight absolute humidity can be 12 g/kg or more, 13 g/kg or more, 14 g/kg or more, 15 g/kg or more, 17 g/kg or more, or 18 g/kg or more, and can be 24 g/kg or less or less than 24 g/kg.

As used herein, the term "film" includes thin films which cannot be carried alone such as coating films, in addition to films which can be carried alone such as wrap films. In the present disclosure, films which can be carried alone, such as wrap films, are referred to as "self-supporting films", and thin films which cannot be carried alone are referred to as "non-self-supporting films", and may be distinguished from each other.

As used herein, "beautifying" or "beautifying method" means applying the cosmetic of the present disclosure to the body surface to beautify and improve the condition of the body surface, or a method of beautifying the condition of the body surface, which differs from methods of surgery, treatment, or diagnosis of humans.

As used herein, "body surface" means body skin surface.

### <<Moisturizing Film Cosmetic>>

The moisturizing film cosmetic of the present disclosure (sometimes referred to simply as "cosmetic") has humidity responsiveness. Such humidity responsiveness can be evaluated by the amount of water evaporation calculated by a water evaporation test. The amount of water evaporation is sometimes referred to as the moisture evaporation rate.

### <Evaluation Method 1>

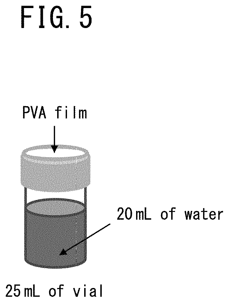

The amount of water evaporation can be calculated as an amount of decrease in water per unit area and unit time by covering the opening of a vial containing 20 mL of water with a measurement sample such as a PVA film cosmetic, and in, for example, a low humidity environment of 40% RH at 23 °C, allowing the sample to stand until an equilibrium state in which the amount of evaporation is substantially constant over time is reached, and thereafter measuring the amount of decrease in water, as shown in FIG. 5.

The cosmetic of the present disclosure can exhibit humidity responsiveness wherein, for example, the amount of water evaporation as measured in a low humidity environment of 40% RH at 23 °C is less than the amount of water evaporation as measured in a high humidity environment of 70% RH at 28 °C when evaluated by the water evaporation test using the amount of water evaporation. In contrast, when an open system or vial is covered with filter paper, the amount of water evaporation as measured in a low humidity environment of 40% RH at 23 °C is higher than the amount of water evaporation measured in a high humidity environment of 70% RH at 28 °C.

In the cosmetic of the present disclosure, specifically, the amount of water evaporation as measured in a low humidity environment of 40% RH at 23 °C can be less than the amount of water evaporation as measured in a high humidity environment of 70% RH at 28 °C by 0.20 mg/cm ²/h or more, 0.25 mg/cm²/h or more, 0.30 mg/cm ²/h or more, 0.40 mg/cm ²/h or more, 0.45 mg/cm ²/h or more, or 0.50 mg/cm ²/h or more. The upper limit thereof can be 1.3 mg/cm ²/h or less, 1.2 mg/cm ²/h or less, 1.0 mg/cm ²/h or less, or 0.95 mg/cm ²/h or less.

Note that when the atmosphere of the measurement environment is, for example, an environment in which the humidity is lower than the environment of 40% RH at 23 °C, the cosmetic of the present disclosure can further suppress the evaporation of moisture. Thus, the differences in the amount of water evaporation and the moisture evaporation ratio, which is described later, tend to increase as the difference in the humidity environment of the atmosphere to be measured increases.

### <Evaluation Method 2>

Humidity responsiveness can also be evaluated using the moisture evaporation ratio (%) obtained as follows. First, the amount of water evaporation 1, which is the amount of water loss per unit area and unit time, is calculated by allowing an open vial containing 20 mL of water to stand in a low humidity environment of 40% RH at 23 °C until the amount of evaporation becomes substantially constant over time, and then measuring the amount of water loss. Next, as shown in FIG. 5, the amount of water evaporation 2, which is the amount of decrease in water per unit area and unit time, is calculated by covering the opening of another vial containing 20 mL of water with a measurement sample of, for example, a PVA film cosmetic, allowing it to stand in a similar environment until it reaches an equilibrium state, and then measuring the amount of water loss. The ratio of amount of water evaporation 2 to the amount of water evaporation 1 can be calculated as a percentage to calculate the moisture evaporation ratio.

The cosmetic of the present disclosure can exhibit humidity responsiveness wherein, for example, the moisture evaporation ratio as measured in a low humidity environment of 40% RH at 23 °C is less than the moisture evaporation ratio as measured in a high humidity environment of 70% RH at 28 °C, when evaluated by the water evaporation test using the moisture evaporation ratio. In contrast, when an open system or vial is covered with filter paper, the amount of water evaporation when measured in a low humidity environment of 40% RH at 23 °C is higher than the amount of water evaporation measured in a high humidity environment of 70% RH at 28 °C.

In the cosmetic of the present disclosure, specifically, the moisture evaporation ratio as measured in a low humidity environment of 40% RH at 23 °C can be less than the moisture evaporation ratio as measured in a high humidity environment of 70% RH at 28 °C by 2.0% or more, 2.5% or more, 3.0% or more, 3.5% or more, or 4.0% or more. The upper limit thereof can be 10% or less, 9.5% or less, 9.0% or less, or 8.5% or less.

### <Polyvinyl Alcohol Film>

The polyvinyl alcohol film constituting the cosmetic of the present disclosure is a film which has humidity responsiveness capable of reducing or suppressing evaporation of moisture from the skin in low humidity environments and which exhibits breathability in high humidity environments. Thus, from the viewpoint of reducing or suppressing evaporation of moisture in low humidity environments, such a film is preferably a film in which openings are not always formed over the entire surface, not a film in which openings are always formed over the entire surface in order to impart breathability, such as a non-woven fabric or a film having physically provided with through holes. However, as long as the polyvinyl alcohol film of the present disclosure can exhibit the above humidity responsiveness, through holes may be partially formed in the film in order to, for example, improve air accumulation when the film is applied.

### (Material)

The material constituting the polyvinyl alcohol film of the present disclosure is not particularly limited as long as the obtained film exhibits humidity responsiveness, and polyvinyl alcohol or derivatives thereof can be used alone or in combination of two or more thereof. Examples of the derivatives of polyvinyl alcohol include modified polyvinyl alcohols, polyvinyl acetate, and polyvinyl butyral. Furthermore, from the viewpoint of humidity responsiveness, it is preferable that the film be obtained from unmodified polyvinyl alcohol and/or modified polyvinyl alcohol.

As the polyvinyl alcohol, for example, fully-saponified polyvinyl alcohols having a degree of saponification of 99% or more, partially-saponified polyvinyl alcohols having a degree of saponification of 90% to less than 99%, or partly-saponified polyvinyl alcohols having a degree of saponification of 70% to less than 90% can be used. These polyvinyl alcohols can be used alone or in combination of two or more thereof. Among these, from the viewpoint of humidity responsiveness, and in particular, the effect of suppressing moisture evaporation in low humidity environments, fully-saponified polyvinyl alcohols and partially-saponified polyvinyl alcohols are preferable, and fully-saponified polyvinyl alcohols are more preferable. Examples of the modification of the polyvinyl alcohol include diol modification, anionic modification, and cationic modification.

From the viewpoint of humidity responsiveness, and in particular, the effect of suppressing moisture evaporation in low humidity environments, unmodified polyvinyl alcohols or diol-modified polyvinyl alcohols are preferable, and unmodified polyvinyl alcohols are more preferable.

Commercially available products can be used for such polyvinyl alcohols. Examples of unmodified fully-saponified polyvinyl alcohols include Gohsenol^{™} NL-05 (manufactured by Mitsubishi Chemical Corporation), KURARAY POVAL PVA-117H (manufactured by TM Kuraray Co., Ltd.), and KURARAY POVAL PVA-124 (manufactured by Kuraray Co., Ltd.). TM Examples of anion-modified fully-saponified polyvinyl alcohols include Gohsenx T-330H (manufactured by Mitsubishi Chemical Corporation). Examples of diol-modified TM fully-saponified polyvinyl alcohols include Nichigo G-Polymer OKS-1089 and Nichigo TM G-Polymer OKS-1109 (both manufactured by Mitsubishi Chemical Corporation).

Examples of the unmodified partially-saponified polyvinyl alcohols include KURARAY TM POVAL PVA-617 (manufactured by Kuraray Co., Ltd.), and examples of diol-modified TM partially-saponified polyvinyl alcohols include Nichigo G-Polymer OKS-8096 (manufactured by Mitsubishi Chemical Corporation).

Examples of unmodified partly-saponified polyvinyl alcohols include Gohsenol^{™} GM-14, Gohsenol^{™} PVA EG-05, Gohsenol^{™} PVA EG-40 (all manufactured by Mitsubishi Chemical Corporation), KURARAY POVAL^{™} PVA-235 (manufactured by Kuraray Co., Ltd.), and examples of cation-modified partly-saponified polyvinyl alcohols include Gohsenol^{™} PVA K-434 (manufactured by Mitsubishi Chemical Corporation).

### (Thickness)

The thickness of the polyvinyl alcohol film is not particularly limited and can be appropriately adjusted in accordance with the location to which the cosmetic of the present disclosure is to be applied, the purpose of use, the sweat gland volume of the user, the amount of water evaporation from the skin, or the balance of humidity responsiveness when combined with a waterproof and water-vapor-permeable layer, which will be described later. For example, the thickness of the polyvinyl alcohol can be 100 µm or less, 70 µm or less, 50 µm or less, 20 µm or less, 15 µm or less, 13 µm or less, 10 µm or less, 7 µm or less, 5 µm or less, 3 µm or less, 1 µm or less, or 0.5 µm or less, and can be 0.01 µm or more, 0.05 µm or more, 0.1 µm or more, 0.2 µm or more, or 0.3 µm or more. In particular, when applied in combination with a waterproof and water-vapor-permeable layer, which will be described later, the thickness of the polyvinyl alcohol film can be reduced to 15 µm or less, 13 µm or less, 10 µm or less, 7 µm or less, 5 µm or less, 3 µm or less, 1 µm or less, 0.7 µm or less, or 0.5 µm or less, and can be 0.01 µm or more, 0.05 µm or more, 0.1 µm or more, 0.2 µm or more, or 0.3 µm or more.

When the polyvinyl alcohol film is a self-supporting film, the thickness of the polyvinyl alcohol film can be defined as an average value calculated by measuring the thickness of an arbitrary part of the film at least 5 times using a high-precision digital micrometer (MDH-25MB, manufactured by Mitutoyo Corporation). If the thickness of the polyvinyl alcohol film to be measured with the high-precision digital micrometer is excessively thin, the thickness of the polyvinyl alcohol film can be determined by the same method using the scanning electron microscope shown below.

When the moisturizing film cosmetic has a laminated structure including, for example, a polyvinyl alcohol film and a waterproof and water-vapor-permeable layer, which is described later, and the waterproof and water-vapor-permeable layer cannot be separated, a scanning electron microscope is used to measure the thickness direction cross-section of such a laminated structure. The average value of the thickness in at least five arbitrary points in the polyvinyl alcohol film in the laminated structure can be defined as the thickness of the polyvinyl alcohol film.

### (Basis Weight)

The polyvinyl alcohol film can also be specified by basis weight instead of thickness. The basis weight of the present disclosure can be defined as the weight (mg) per cm² of the film. The basis weight of the polyvinyl alcohol film can be, for example. 0.0010 mg/cm ² or more, 0.0060 mg/cm² or more, 0.010 mg/cm² or more, 0.025 mg/cm ² or more, 0.035 mg/cm ² or more, 0.050 mg/cm² or more, 0.070 mg/cm² or more, 0.10 mg/cm ² or more, 0.15 mg/cm ² or more, 0.30 mg/cm ² or more, or 0.45 mg/cm² or more, and can be 12.0 mg/cm ² or less, 8.5 mg/cm ² or less, 6.0 mg/cm ² or less, 4.5 mg/cm ² or less, 2.5 mg/cm² or less, 2.0 mg/cm ² or less, 1.5 mg/cm ² or less, 1.2 mg/cm ² or less, 1.0 mg/cm ² or less, 0.80 mg/cm ² or less, 0.60 mg/cm ² or less, 0.35 mg/cm ² or less, 0.15 mg/cm ² or less, 0.10 mg/cm ² or less, 0.085 mg/cm² or less, or 0.060 mg/cm ² or less.

### (Optional Components)

Various components can be appropriately blended in the polyvinyl alcohol film of the present disclosure as long as the effects of the present invention are not influenced thereby. Examples of components include moisturizers, polymers (for example, carboxyvinyl polymers, (N,N-dimethylacrylamide-2-acrylamide dimethylpropanesulfonic acid) crosslinked copolymers, and copolymers of 2-methacloyloxy phosphorylcholine (MPC) and n-butylmethacrylate (BMA)), rubbers, elastomers, pressure-sensitive adhesives, adhesives, pressure-sensitive adhesive modifiers, adhesive modifiers, UV absorbers, antioxidants, preservatives, antioxidant aids, crosslinkers, light diffusers, fillers, skin nutritional supplements, vitamins, various chemicals applicable to pharmaceuticals, quasi-drugs, cosmetics and the like, organic powders, colorants, dyes, pigments, and fragrances. These optional components can be used alone or in combination of two or more thereof. Since glycerin, which is a moisturizer, may increase breathability in low humidity environments, moisturizers other than glycerin, such as dipropylene glycol and POE(14)POP(7)dimethyl ether, are preferable. POE means polyoxyethylene, and POP means polyoxypropylene.

### <Waterproof and Water-vapor-permeable Layer>

The moisturizing film cosmetics of the present disclosure may further comprise a waterproof and water-vapor-permeable layer. The waterproof and water-vapor-permeable layer is a layer having waterproofness and breathability, and is a layer which does not exhibit humidity responsiveness by itself.

As shown in FIG. 3, for example, when the polyvinyl alcohol film 301 is thin and the amount of moisture on the skin is large, gaps 307 through which the moisture passes are formed even in low humidity environments, and the moisture from the skin may evaporate. In such a case, as shown in FIG. 4, by applying a waterproof and water-vapor-permeable layer 409 between the polyvinyl alcohol film 401 and the skin, it is possible to suppress the formation of gaps 407 through which the moisture passes, whereby the moisturizing property can be secured.

Furthermore, when the polyvinyl alcohol film is thick, the rigidity of the cosmetic itself increases, making it difficult to apply to the skin or making it look unattractive. Even if the waterproof and water-vapor-permeable layer is relatively thin, when combined with a polyvinyl alcohol film, the cosmetic provided with these can provide sufficient humidity responsiveness, whereby the overall thickness of cosmetics can be reduced. As a result, a cosmetic obtained from a combination of a waterproof and water-vapor-permeable layer and a thin polyvinyl alcohol film can have improved skin applicability and appearance as compared with a cosmetic having a thick polyvinyl alcohol film.

Since the surface opposite the body surface of the polyvinyl alcohol film is the surface used to adjust breathability with the external environment, it is preferable that neither a waterproof and water-vapor-permeable layer nor any additional layer be formed on such a surface. Thus, when the waterproof and water-vapor-permeable layer is applied to the polyvinyl alcohol film in advance and used, it is preferable that the waterproof and water-vapor-permeable layer be configured so as to be arranged closer to the body surface side than the polyvinyl alcohol film.

The waterproof and water-vapor-permeable layer may have a single layer structure or a laminated structure. The waterproof and water-vapor-permeable layer may be in the form of a self-supporting film or a non-self-supporting film. Further, the waterproof and water-vapor-permeable layer may be configured so as to be applied to the entire surface or a part of the polyvinyl alcohol film.

### (Material)

The waterproof and water-vapor-permeable layer is not particularly limited as long as it is a layer capable of exhibiting the above humidity responsiveness when combined with a polyvinyl alcohol film. Examples of the waterproof and water-vapor-permeable layer include at least one selected from an oil layer, a polylactic acid layer, and an artificial skin.

Examples of the oil layer material include polar oils such as ester oils and non-polar oils such as hydrocarbon oils. Among these, non-polar oils are preferable from the viewpoint of humidity responsiveness.

Examples of non-polar oils include petroleum jelly, liquid paraffin, tetraisobutane, hydrogenated polydecene, olefin oligomer, isododecane, isohexadecane, squalane, polybutene, hydrogenated polybutene, polyisobutene, and hydrogenated polyisobutene. These can be used alone or in combination of two or more thereof. Among these, petroleum jelly and liquid paraffin are preferable from the viewpoint of humidity responsiveness.

As the material constituting artificial skin, for example, rubbers or elastomers such as urethane rubber, olefin rubber, silicone rubber, acrylic rubber, natural rubber, and α-gel can be used. These can be used alone or in combination of two or more thereof. Among these, silicone rubber is preferable from the viewpoint of flexibility and humidity responsiveness.

### (Thickness)

The thickness of the waterproof and water-vapor-permeable layer is not particularly limited and can be appropriately adjust in accordance with the location to which the waterproof and water-vapor-permeable layer is to be applied, the purpose of use, the sweat gland volume of the user, the amount of water evaporation from the skin, or the balance of humidity responsiveness when combined with the above polyvinyl alcohol film.

### (Basis Weight)

The waterproof and water-vapor-permeable layer can be specified by, for example, basis weight. The basis weight of the waterproof and water-vapor-permeable layer can be, for example, 1.0 mg/cm ² or more, 1.2 mg/cm ² or more, or 1.4 mg/cm ² or more, and can be 2.0 mg/cm ² or less, 1.8 mg/cm ² or less, or 1.6 mg/cm ² or less.

### (Optional Components)

Various components can be appropriately blended in the waterproof and water-vapor-permeable layer as long as the effects of the present invention are not influenced thereby. Examples of components include moisturizers, polymers, rubbers, elastomers, pressure-sensitive adhesives, adhesives, pressure-sensitive adhesive modifiers, adhesive modifiers, UV absorbers, antioxidants, preservatives, antioxidant aids, light diffusers, fillers, skin nutritional supplements, vitamins, various chemicals applicable to pharmaceuticals, quasi-drugs, cosmetics and the like, organic powders, colorants, dyes, pigments, and fragrances. These optional components can be used alone or in combination of two or more thereof.

### <Optional Constituent Layers>

The moisturizing film cosmetic of the present disclosure may comprise optional constituent layers as long as the effects of the present invention are not influenced thereby. Examples of such constituent layers include pressure-sensitive adhesive layers having breathability (for example, a pressure-sensitive adhesive layer having through holes), protective films having peeling performance, and release sheets, and these can be used alone or in combination of two or more thereof. For example, a protective film can be applied to one side of the polyvinyl alcohol film and/or a release sheet can be applied to the other side. The surfaces of the protective film and the release sheet may be subjected to release treatment with, for example, a silicone. Furthermore, the peeling performances of the protective film and the release sheet with respect to the polyvinyl alcohol film may be the same or different. From the viewpoint of ease of peeling from the polyvinyl alcohol film, it is preferable that the peeling performances thereof be different.

The materials of the optional constituent layers are not particularly limited, and example thereof include, from the viewpoint of handleability, polyolefins such as polyethylene and polypropylene, polyesters such as polyethylene terephthalate, cotton, and paper. The optional constituent layers may be in the form of a film, or may be in the form of a non-woven fabric, knitted fabric, or woven fabric.

### <<Production Method of Moisturizing Film Cosmetic>>

The moisturizing film cosmetic of the present disclosure can be produced, for example, using a commercially available polyvinyl alcohol film having a predetermined thickness, or can be produced using a composition for producing a polyvinyl alcohol film.

As the production method using the composition for producing a polyvinyl alcohol film, for example, the composition is applied onto a release sheet using an arbitrary means, such as offset printing, gravure coating, bar coating, knife coating, spray coating, and other known coating means, and optionally, for example, a drying step, a cross-linking step, a waterproof and water-vapor-permeable layer application step, a bonding step of constituent layers such as a protective film, and a winding step can be adopted for continuous preparation.

The cosmetic of the present disclosure can also be prepared non-continuously using the composition for producing a polyvinyl alcohol film. The cosmetic of the present disclosure can be prepared by, for example, adding a predetermined amount of such composition to a container or a molding mold and drying to prepare a polyvinyl alcohol film, and if necessary, for example, applying a waterproof and water-vapor-permeable layer, protective film, or release sheet to the obtained film. Alternatively, the cosmetic may be formed directly on the body surface by placing the composition into, for example, a tube container or a spray container, and from such a container, applying the composition to the body surface, or applying the composition onto a waterproof and water-vapor-permeable layer previously applied to the body surface and dried.

In the moisturizing film cosmetic, the size and shape of the cosmetic to be applied can be adjusted using known cutting means such as half-cutting or laser-cutting depending on the location of application.

### <Composition for Producing Polyvinyl Alcohol Film>

The polyvinyl alcohol film constituting the cosmetic of the present disclosure can be prepared using a composition for producing a polyvinyl alcohol film. Such a composition can be prepared by appropriately mixing the above polyvinyl alcohol, water, and the following optional components.

### (Optional Components)

Various components can be appropriately blended in the composition for producing a polyvinyl alcohol film of the present disclosure as long as the effects of the present invention are not influenced thereby. Examples of components include lower alcohols, polyhydric alcohols, higher alcohols, oils, various extracts, anionic surfactants, cationic surfactants, amphoteric surfactants, nonionic surfactants, moisturizers, polymers, rubbers, elastomers, pressure-sensitive adhesives, adhesives, pressure-sensitive adhesive modifiers, adhesive modifiers, UV absorbers, antioxidants, preservatives, antioxidant aids, cross-linking agents, light diffusers, fillers, gelling agents (thickeners), skin nutrients, vitamins, various chemicals applicable to pharmaceuticals, quasi-drugs, cosmetics and the like, organic powders, colorants, dyes, pigments, and fragrances. These optional components can be used alone or in combination of two or more thereof.

### <<Cosmetic Kit>

The moisturizing film cosmetic of the present disclosure can be provided as a cosmetic kit comprising a polyvinyl alcohol film constituting such a cosmetic or a composition for producing the polyvinyl alcohol film.

<Optional Members>

The cosmetic kit may have other optional members. Examples of such optional members include a member to which the above waterproof and water-vapor-permeable layer can be applied, a cutter, scissors, and a mirror. These optional members can be provided in the cosmetic kit alone or in combination of two or more thereof.

### <<Beautifying Method with Moisturizing Film Cosmetic>>

The moisturizing film cosmetic of the present disclosure are carried out by applying such cosmetic to the body surface. Cosmetics applied to the skin can reduce or suppress the evaporation of moisture from the skin in low humidity environments, and can sufficiently moisturize the skin even in low humidity environments. As a result, the function of producing moisturizing ingredients created by the skin itself is improved, and turnover disorders in the horny layer are also improved, whereby skin troubles such as rough skin are less likely to occur, and the beautifying effect can be enhanced.

The means for applying the cosmetic to the body surface is not limited, and for example, the polyvinyl alcohol film constituting the cosmetic may be attached to the body surface in the form of a self-supporting film, or may be carried out by applying the composition for producing a polyvinyl alcohol film, which will be described later, to the body surface.

As means for attaching the polyvinyl alcohol film to the body surface in the form of a self-supporting film, for example, when the polyvinyl alcohol film has a pressure-sensitive adhesive layer having breathability, it can be attached to the body surface via such a pressure-sensitive adhesive layer. Since the polyvinyl alcohol film exhibits pressure-sensitive adhesiveness when it comes into contact with water, the film may be attached to the location of the body surface to which the film is to be applied after applying water or a skin lotion, and alternatively, water or a skin lotion may be spread on the polyvinyl alcohol film, and the film may then be applied to the body surface.

As the means for applying the composition for producing a polyvinyl alcohol film to the body surface, for example, the composition may be sprayed from a spray container containing the composition so as to form a film, and alternatively, the composition may be placed into a container having no spray function, an appropriate amount of the composition may be collected from such a container on a finger or the like, and the composition may be spread on the body surface.

The application of the polyvinyl alcohol film to the body surface may be carried out after the waterproof and water-vapor-permeable layer is applied to the body surface. In this case, the application of the waterproof and water-vapor-permeable layer to the body surface may be carried out, for example, by attaching in the form of a self-supporting film, and alternatively, it may be carried out by applying waterproof and water-vapor-permeable layer constituent components to the body surface. The application of the polyvinyl alcohol film to the waterproof and water-vapor-permeable layer may be carried out in the same manner as the means of applying the polyvinyl alcohol film to the body surface, and alternatively, when the waterproof and water-vapor-permeable layer itself has a pressure-sensitive adhesiveness, the polyvinyl alcohol film may be attached utilizing this pressure-sensitive adhesiveness.

As means for attaching the waterproof and water-vapor-permeable layer to the body surface in the form of a self-supporting film, for example, when the waterproof and water-vapor-permeable layer itself has pressure-sensitive adhesiveness, the waterproof and water-vapor-permeable layer in this form can be directly attached to the body surface. When the waterproof and water-vapor-permeable layer itself lacks pressure-sensitive adhesiveness, for example, such a form of waterproof and water-vapor-permeable layer can be attached to the body surface via a pressure-sensitive adhesive layer having breathability or another waterproof and water-vapor-permeable layer having pressure-sensitive adhesiveness.

The application of the waterproof and water-vapor-permeable layer constituent components onto the body surface can be carried out by preparing a composition containing the various components constituting the waterproof and water-vapor-permeable layer and carrying out the same method as the above composition for producing a polyvinyl alcohol film.

In the application of the polyvinyl alcohol film to the body surface, after applying the waterproof and water-vapor-permeable layer to one side of the polyvinyl alcohol film, the polyvinyl alcohol film may be applied to the body surface via this waterproof and water-vapor-permeable layer. In the application of the waterproof and water-vapor-permeable layer to the polyvinyl alcohol film, application to the polyvinyl alcohol film utilizing the pressure-sensitive adhesiveness can be carried out when the waterproof and water-vapor-permeable layer itself has pressure-sensitive adhesiveness. Furthermore, when the waterproof and water-vapor-permeable layer lacks pressure-sensitive adhesiveness, the waterproof and water-vapor-permeable layer can be applied to the polyvinyl alcohol film via a pressure-sensitive adhesive layer having breathability or another waterproof and water-vapor-permeable layer having pressure-sensitive adhesiveness. In this case, a water-vapor-permeable pressure-sensitive adhesive layer or another waterproof and water-vapor-permeable layer having pressure-sensitive adhesiveness is also applied to the surface of the waterproof and water-vapor-permeable layer on the body surface side, and alternatively, a polyvinyl alcohol film having a non-pressure-sensitive adhesive waterproof and water-vapor-permeable layer can be applied to the body surface by applying a water-vapor-permeable pressure-sensitive adhesive layer or another waterproof and water-vapor-permeable layer having pressure-sensitive adhesiveness to the body surface.

### <<Application Location of Moisturizing Film Cosmetic>>

The moisturizing film cosmetic of the present disclosure can be applied to any part of the body on the surface of the skin, i.e., on the body surface. For example, it can be appropriately attached to the skin surface of face (lips, eyes, nose, cheeks, or forehead), neck, ears, hands, arms, legs, feet, chest, abdomen, or back. The skin also includes the nails in which the keratin of the epidermis of the skin is changed and hardened.

### EXAMPLES

The present invention will be described in further detail by way of the following Examples. However, the present invention is not limited thereto.

### <<Examples 1 to 3 and Comparative Examples 1 and 2>>

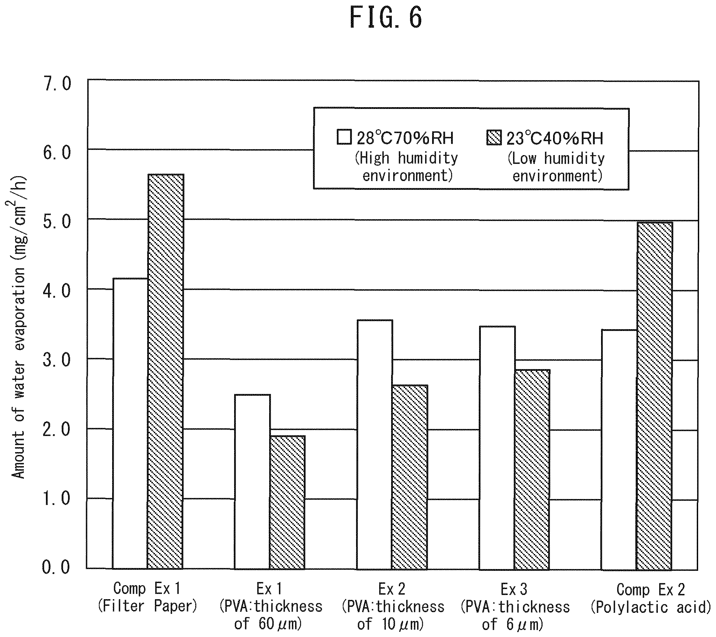

Regarding the polyvinyl alcohol (PVA) films obtained by the production methods described in Examples 1 to 3 and the filter paper (Comparative Example 1) and polylactic acid film (Comparative Example 2) used for comparison, humidity responsiveness was evaluated by the water evaporation test shown below. The results are shown in Table 1 and FIG. 6. As a reference example, Table 1 shows the results of an open system sample.

### <Water Evaporation Test>

The circular opening (radius: 0.7 cm, area: 1.54 cm²) of a vial containing 20 mL of water was covered with each measurement sample, and the vial was allowed to stand in a constant temperature bath under predetermined temperature and humidity conditions. After allowing to stand for approximately 2 to 3 days until the amount of evaporation became substantially constant with respect to time, the amount of decrease in water in the vial was measured, and the amount of water evaporation, which is the amount of decrease in water per unit area and unit time, was calculated. The test was carried out in two environments; a low humidity environment of 40% RH at 23 °C and a high humidity environment of 70% RH at 28 °C.

### <Example 1>

A composition for producing a polyvinyl alcohol film was prepared by mixing KURARAY TM POVAL PVA-117H (manufactured by Kuraray Co., Ltd.), which is an unmodified fully-saponified polyvinyl alcohol, and ion-exchanged water in a 10 mL vial so as to achieve a polyvinyl alcohol concentration of 5.0% by mass, followed by stirring. Next, a polyvinyl alcohol film having a thickness of approximately 60 µm and a basis weight of approximately 7.6 mg/cm ², serving as the measurement sample, was prepared by adding 3.0 g of the obtained 5.0 mass% aqueous solution composition into a substantially circular petri dish having an area of 19.6 cm² and drying.

### <Example 2>

A polyvinyl alcohol film serving as the measurement sample was prepared in the same manner as Example 1, except that the concentration of polyvinyl alcohol was changed to 1.0 mass%. The thickness of the obtained film was approximately 10 µm, and the basis weight was approximately 1.5 mg/cm ².

### <Example 3>

A polyvinyl alcohol film serving as the measurement sample was prepared in the same manner as Example 1, except that the concentration of polyvinyl alcohol was changed to 0.5 mass%. The thickness of the obtained film was approximately 6 µm, and the basis weight was approximately 0.76 mg/cm ².

### <Comparative Example 1>

ADVANTEC filter paper #133 having a thickness of 250 µm and a basis weight of 14 mg/cm ² was used as a measurement sample of Comparative Example 1.

### <Comparative Example 2>

A polylactic acid film having a thickness of approximately 200 nm and a basis weight of approximately 0.025 mg/cm ² was prepared by the spin-coating method using a 0.1 mass% polylactic acid solution prepared using tetrahydrofuran. A stack of two polylactic acid films was used as the measurement sample of Comparative Example 2.

### <Reference Example 1>

As a reference example, an open sample with no lid on the opening of the vial was used.

**[Table 1]**

| | | Moisture Evaporation Amount (mg/cm²/h) | | |
|---|---|---|---|---|
| | | 23 °C/40% RH (Low humidity) | 28 °C/70% RH (High humidity) | (High humidity) - (Low humidity) |
| Ex 1 | PVA film having thickness of 60 µm | 1.90 | 2.50 | 0.60 |
| Ex 2 | PVA film having thickness of 10 µm | 2.64 | 3.57 | 0.93 |
| Ex 3 | PVA film having thickness of 6 µm | 2.86 | 3.48 | 0.62 |
| Comp Ex 1 | Filter paper | 5.64 | 4.15 | -1.49 |
| Comp Ex 2 | Polylactic acid film | 4.97 | 3.44 | -1.53 |
| Ref Ex 1 | Open System | 35.1 | 23.9 | -11.2 |

### <Results>

As is clear from Table 1 and FIG. 6, in the case of the filter paper of Comparative Example 1 and the polylactic acid film of Comparative Example 2, the amount of evaporation of moisture was larger in the low humidity environment than in the high humidity environment, and humidity responsiveness was not exhibited. Conversely, in the polyvinyl alcohol films of Examples 1 to 3, the amount of water evaporation in the low humidity environment was clearly lower than the amount of water evaporation in the high humidity environment, and it could be confirmed that the polyvinyl alcohol film exhibited humidity responsiveness.

### <<Examples 4 and 5 and Comparative Example 3>>

An evaluation of the amount of water evaporation was performed on human skin, and the results are summarized in Table 2. The amount of water evaporation was measured using a VAPO METER manufactured by Keystone Scientific K.K. in two environments; a low humidity environment of 40% RH at 23 °C and a high humidity environment of 70% RH at 28 °C.

### <Example 4>

The polyvinyl alcohol film (thickness: approximately 10 µm) obtained in Example 2 was attached to the skin surface on the inside the left arm of a human.

### <Example 5>

Petroleum jelly (Sun White P-1, manufactured by Nikko Rica Corporation) was applied to the skin surface on the inside of the left arm of a human at a ratio of about 1.5 mg/cm ² to form a waterproof and water-vapor-permeable layer, and the polyvinyl alcohol film (thickness of about 10 µm) obtained in Example 2 was attached thereto.

### <Comparative Example 3>

The amount of water evaporation of bare skin was measured.

**[Table 2]**

| | | Moisture Evaporation Amount(g/m ²/h) | | |
|---|---|---|---|---|
| | | 23 °C/40% RH (Low humidity) | 28 °C/70% RH (High humidity) | (High humidity) - (Low humidity) |
| Ex 4 | PVA film | 5.6 | 6.2 | 0.6 |
| Ex 5 | PVA film/waterproof and water-vapor-permeable layer | 2.9 | 4.1 | 1.2 |
| Comp Ex 3 | Bare skin | 5.7 | 4.5 | -1.2 |

### <Results>

As is clear from Table 2, for bare skin itself, the amount of evaporation of moisture was larger in the low humidity environment than in the high humidity environment. Conversely, in the case of Examples 4 and 5, in which a polyvinyl alcohol film was applied, in the high humidity environment, there was no sticky uncomfortable feeling, and the amount of water evaporation in the low humidity environment was maintained lower than the amount of water evaporation in the high humidity environment, whereby it could be confirmed that the moisturizing performance of the skin in the low humidity environment was improved. Note that though the amount of water evaporation of Example 4 was larger than the amount of water evaporation in Comparative Example 3 in the high humidity environment, it is considered that this is because the measurement location of the bare skin of Comparative Example 3 and the location where the PVA film of Example 4 was applied to the bare skin were different, i.e., the amount of water evaporation from the bare skin at each measurement point was different.

### DESCRIPTION OF REFERENCE SIGNS

- 100, 200, 300, 400: moisturizing film cosmetic
- 101, 201, 301, 401: polyvinyl alcohol film
- 103, 203, 303, 403: polyvinyl alcohol molecules
- 105, 205, 305, 405: moisture
- 107, 207, 307, 407: gap through which moisture passes
- 408: moisture passing through waterproof and water-vapor-permeable layer
- 409: waterproof and water-vapor-permeable layer

## Claims

1. A moisturizing film cosmetic, comprising a polyvinyl alcohol film.

2. The cosmetic according to claim 1, wherein the thickness of the polyvinyl alcohol film is 100 µm or less.

3. The cosmetic according to claim 1 or 2, wherein the polyvinyl alcohol film contains an unmodified polyvinyl alcohol.

4. The cosmetic according to any one of claims 1 to 3, further comprising a waterproof and water-vapor-permeable layer arranged closer to a body surface side than the polyvinyl alcohol film.

5. The cosmetic according to claim 4, wherein the waterproof and water-vapor-permeable layer is at least one selected from an oil layer containing at least one of petroleum jelly and liquid paraffin, a polylactic acid layer, and an artificial skin.

6. The cosmetic according to any one of claims 1 to 5, wherein, when evaluated by a water evaporation test, an amount of water evaporation as measured in a 40% RH environment at 23 °C is less than an amount of water evaporation as measured in a 70% RH environment at 28 °C.

7. A composition for producing a polyvinyl alcohol film of the cosmetic according to any one of claims 1 to 6, comprising a polyvinyl alcohol and water.
